(19) Europäisches Patentamt
European Patent Office
Office européen des brevets

(11) Publication number : 0 365 183 B1

(12) EUROPEAN PATENT SPECIFICATION

(45) Date of publication of patent specification : **10.02.93 Bulletin 93/06**

(51) Int. Cl.$^5$ : C07C 311/21, C07C 311/29, A61K 31/18

(21) Application number : **89310242.6**

(22) Date of filing : **06.10.89**

# (54) Biologically active compounds.

(30) Priority : **10.10.88 GB 8823731**

(43) Date of publication of application : **25.04.90 Bulletin 90/17**

(45) Publication of the grant of the patent : **10.02.93 Bulletin 93/06**

(84) Designated Contracting States : **AT BE CH DE ES FR GB GR IT LI LU NL SE**

(56) References cited :
**DE-A- 3 700 408**
**US-A- 3 629 320**
**US-A- 4 443 477**
**JOURNAL OF THE CHEMICAL SOCIETY, Perkin Transactions 1, September 1985 (no. 9), London, GB; I.G. COUTTS et al.:"Spirodienones. Part 5. The synthesis and Reactions of N-Sulphonylcyclohexadienimines", pages 1829-1836**
**IDEM**

(73) Proprietor : **SMITH KLINE & FRENCH LABORATORIES LIMITED**
**Mundells**
**Welwyn Garden City Hertfordshire, AL7 1EY (GB)**

(72) Inventor : **Cooper, David Gwyn**
**SmithKliney Pharmaceuticals, The Frythe**
**Welwyn Hertfordshire AL6 9AR (GB)**
Inventor : **Gribble, Andrew Derrick**
**SmithKline Pharmaceuticals, The Frythe**
**Welwyn Hertfordshire AL6 9AR (GB)**

(74) Representative : **Giddings, Peter John, Dr. et al**
**Smith Kline & French Laboratories Ltd.**
**Corporate Patents Mundells**
**Welwyn Garden City Hertfordshire AL7 1EY (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 365 183 B1

## Description

The present invention relates to a class of phenylalkanoic acid compounds containing a sulphonamido group which have activity as thromboxane $A_2$ antagonists, to the use of the compounds in medicine, to pharmaceutical compositions containing them and to methods for their preparation.

Thromboxane $A_2$ ($TXA_2$) is a potent vasoconstricting and platelet aggregating agent which is formed in platelets and other tissues as a product of the "arachidonic acid cascade". $TXA_2$ is produced by the thromboxane synthetase catalysed conversion of prostaglandin $H_2$ ($PGH_2$) which in turn is produced, via the intermediacy of prostaglandin $G_2$ ($PGG_2$), by the action of cyclooxygenase on arachidonic acid. The potency of $TXA_2$ is such that very small amounts can trigger serious biological consequences and it has been implicated in mediating pathophysiological actions in severe disorders such as circulatory shock and myocardial ischaemia.

One method of inhibiting the effects of thromboxane $A_2$ is through the selective antagonism of $TXA_2/PGH_2$ at the receptor level and various compounds have been reported as $TXA_2$ receptor antagonists, see for example U.S. 4,536,510. U.S. 4,443,477 discloses sulphonamidophenylcarboxylic acid compounds having $TXA_2$ antagonist activity.

The present invention provides a compound of the formula (I):

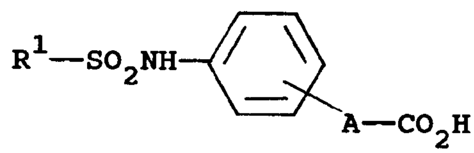

(I)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy,nitro and trifluoromethyl; and A is an acyclic hydrocarbon group having from 2 to 8 linear carbon atoms, particularly 2 to 6 carbon atoms and preferably 2 to 4 carbon atoms provided that when A is CH=CH, $(CH_2)_2$, or $(CH_2)_3$, $R^1$ is other than 4-methylphenyl.

By linear carbon atoms is meant those carbon atoms extending in an unbranched chain between the phenylene ring and the carboxyl group. Each linear carbon atom can be substituted by one or two $C_{1-2}$alkyl substituents, preferably methyl substituents. It is preferred that the total number of carbon atoms in the group A does not exceed 10.

Particular groups A are $(CH_2)_3$, $(CH_2)_4$, $(CH_2)_2C(CH_3)_2$ and $(CH_2)_3C(CH_3)_2$, a preferred group being $(CH_2)_3$.

$R^1$ is suitably an unsubstituted phenyl group or, more suitably a phenyl group substituted by one or two substituents, preferably one of which is located at the 4-position of the phenyl ring.

Particular substituents are bromine, chlorine, methyl, methoxy, trifluoromethyl and nitro, particularly preferred substituents being bromine and chlorine.

Preferred groups $R^1$ include 4-chlorophenyl and 4-bromophenyl.

The group A-$CO_2$H can be ortho, meta or para with respect to the group $R^1SO_2NH$ but particularly it is in the para position.

Preferred compounds of the present invention are
4-[4-(phenylsulphonamido)phenyl]butyric acid;
4-[4-(4-chlorophenylsulphonamido)phenyl]butyric acid;
4-[4-(4-bromophenylsulphonamido)phenyl]butyric acid;
4-[4-(4-methylphenylsulphonamido)phenyl]butyric acid;
4-[4-(4-methoxyphenylsulphonamido)phenyl]butyric acid;
2,2-dimethyl-4-[4-(4-methylphenylsulphonamido)phenyl]-butyric acid;
2,2-dimethyl-4-[4-(4-chlorophenylsulphonamido)phenyl]-butyric acid;
2,2-dimethyl-4-[4-(4-bromophenylsulphonamido phenyl]-butyric acid;
2,2-dimethyl-4-[4-phenylsulphonamido)phenyl]butyric acid; and
2,2-dimethyl-4-[4-(4-methoxyphenylsulphonamido)phenyl]butyric acid;
and pharmaceutically acceptable salts thereof.

Compounds of the formula (I) can form carboxylate salts and salts of the sulphonamide group.

Examples of carboxylate salts are alkali metal, alkaline earth metal and ammonium salts. Alkali and alkaline earth metal salts typically are formed by interaction of a carboxylic acid with a metal alkoxide or hydroxide whereas ammonium salts typically are formed by interaction of the carboxylic acid with the appropriate amine or the appropriate ammonium hydroxide.

It is preferred that the salts are pharmaceutically acceptable, although non-pharmaceutical salts are also

within the scope of the invention. Such salts can be converted into pharmaceutically acceptable salts or into the corresponding free base or free acid.

Where the compounds of formula (I) exist as solvates, for example hydrates and alcoholates, such forms are also within the scope of the invention.

Compounds of the formula (I) can be prepared by the reaction of a compound of the formula (II):

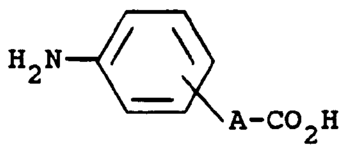

(II)

or a carboxylate salt, amide or ester thereof, with a compound $R^1SO_2L$ where L is a leaving group displaceable by amino.

Examples of leaving groups L are the halogens, particularly chlorine.

The reaction of compounds of the formula (II) with compounds of the formula $R^1SO_2L$ can be conducted under conditions known for the preparation of analogous sulphonamides. Thus, for example, the reaction can be conducted in a solvent, for example benzene, toluene or a polar solvent such as acetone, acetonitrile, a halogenated hydrocarbon such as dichloromethane or a basic solvent such as pyridine, with heating where required, for example at the reflux temperature of the solvent. Where the solvent is non-basic the reaction typically is conducted in the presence of a base such as pyridine or a trialkylamine such as triethylamine.

Alternatively, the reaction can be conducted under Schotten-Baumann conditions, i.e. the reactants are stirred or shaken together in the presence of an aqueous alkali such as dilute sodium hydroxide.

Compounds of the formula (II) can be prepared from a compound of the formula (III):

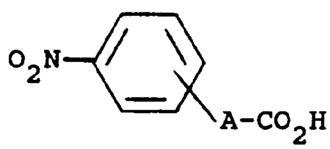

(III)

by treatment with an appropriate reducing agent, for example by hydrogenating over a transition metal catalyst such as palladium on charcoal, or by treatment with hydrazine in the presence of palladium on charcoal. Suitable solvents for use in such reactions are $C_{1-4}$alkanols such as methanol and ethanol and typically the reaction is conducted at approximately ambient temperature.

Compounds of the formula (I) are useful in the treatment of diseases in which $TXA_2$ is a factor. Thus they would be useful in the treatment of disorders in which aggregation of blood platelets and vasoconstriction play a part.

Particular clinical indications in which the present compounds would be of interest include the treatment or management of post myocardial infarction, coronary thromboses (e.g. in combination with tissue plasminogen activator and other thrombolytics), unstable angina, transient ischaemia, coronary artery bypass grafts, cardiac valve replacement and peripheral and vascular grafts including for example renal transplants.

The compounds of the formula (I) can be administered as the pure compound but it is more usual to administer them as part of a pharmaceutical composition in association with a carrier and one or more excipients.

In a further aspect, therefore, the present invention provides a pharmaceutical composition comprising a compound of the formula (IA):

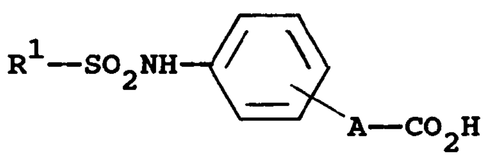

(IA)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy, nitro and trifluoromethyl; and A is an acyclic hydrocarbon group having from 2 to 4 linear carbon atoms, and a pharmaceutically acceptable carrier.

The compositions can be administered in standard manner, for example orally, parenterally, transdermally,

rectally, via inhalation or via buccal administration.

Compounds of formula (IA) and their pharmaceutically acceptable salts which are active when given orally or via buccal administration can be formulated as syrups, tablets, capsules and lozenges. A syrup formulation will generally consist of a suspension or solution of the compound or salt in a liquid carrier for example, ethanol, glycerine or water with a flavouring or colouring agent. Where the composition is in the form of a tablet, any pharmaceutical carrier routinely used for preparing solid formulations may be used. Examples of such carriers include magnesium stearate, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinylpyrrolidone, lecithin, arachis oil, or sesame oil. Such compositions can be administered, for example, by bolus injection or by infusion.

A typical suppository formulation comprises a compound of formula (IA) or a pharmaceutically acceptable salt thereof which is active when administered in this way, with a binding and/or lubricating agent, for example polymeric glycols, gelatins, cocoa-butter or other low melting vegetable waxes or fats.

Typical transdermal formulations comprise a conventional aqueous or non-aqueous vehicle, for example a cream, ointment, lotion or paste or are in the form of a medicated plaster, patch or membrane.

Typical compositions for inhalation are in the form of a solution, suspension or emulsion that may be administered in the form of an aerosol using a conventional propellant such as dichlorodifluoromethane or trichlorofluoromethane.

Preferably the composition is in unit dosage form, for example a tablet, capsule or metered aerosol dose, so that the patient may administer to himself a single dose.

Each such dosage unit suitably contains from 1 mg to 1 g, preferably from 5 mg to 500 mg, e.g. 100 mg or 200 mg, of a compound of the formula (IA) or a pharmaceutically acceptable salt thereof calculated as the compound itself.

A typical daily dosage regimen is 10 mg to 1 g for an average human weighing approximately 70 kg, administered in 1 to 4 dosage units, preferably 1 or 2.

The compositions of this invention, in addition to containing a compound of the formula (IA) can also contain other agents; for example one or more agents chosen from phosphodiesterase inhibitors, hypolipidaemic agents, platelet aggregation inhibitors, vasodilators, β-adrenergic receptor blockers, ACE inhibitors, tissue plasminogen activator and other thrombolytics, and antiarrhythmics.

The compositions of the present invention are prepared by bringing the active constituent into association with a pharmaceutically acceptable carrier and optionally other excipients and ingredients as defined above.

As indicated above, compounds of the formula (IA) have biological activity that is indicative of an ability to antagonise $TXA_2$ receptors. The $TXA_2$ activity has been demonstrated in the human platelet binding assay.

The platelet binding assay used was essentially the method described by Mais et al, J. Pharm. Exp. Ther., 1985, 235(3), 729-734 where [$^{125}$I]PTA-OH was used as the receptor ligand.

The $IC_{50}$ values represent the concentration which produces a 50% inhibition of specific [$^{125}$I]PTA-OH binding.

The compounds of Examples 1 to 4 had $IC_{50}$ values in the range of 0.02 to 0.6 μM in the platelet binding assay.

The following Examples are illustrative of the invention.

In the Examples, all temperatures are in °C. Melting points are uncorrected and were obtained in an open capillary tube using a Büchi 510 Melting Point Apparatus.

## Example 1

### A. 4-(4-Aminophenyl)butyric acid

A mixture of 4-(4-nitrophenyl)butyric acid (3g, 14mmol) (Aldrich Chemical Company Ltd.) and 10% palladium on charcoal catalyst (0.3g) in ethanol (100ml) was submitted to gaseous hydrogen at 3.4 x $10^5$ N/$m^2$ (50 p.s.i.), 20°C, for 30 minutes. The mixture was then filtered and concentrated. The resulting solid residue was recrystallised from isopropanol to give 4-(4-aminophenyl)butyric acid (1.9g, 76%) mp. 125-127°C.

B. 4-[4-(Phenylsulphonamido)phenyl]butyric acid

To a solution of 4-(4-aminophenyl)butyric acid (1g, 5.6mmol) in dry acetone (30ml) was added dropwise a solution of benzenesulphonyl chloride (0.99g) and pyridine (1.1g) in dry acetone (10ml). The mixture was heated at reflux for 3 hours, then concentrated in vacuo. The resulting oil was dissolved in dichloromethane (100ml) and the solution was washed with 2N aqueous HCl (2 x 100ml), followed by water (2 x 100ml). The dichloromethane solution was extracted with 10% aqueous NaOH (2 x 50ml), followed by water (2 x 50ml), and these extracts were combined and stirred with dichloromethane (100ml) while conc. HCl was added to adjust the pH of the mixture to 5. The layers were separated and the organic solution was dried ($MgSO_4$) and concentrated in vacuo. The resulting solid was recrystallised from isopropanol to give 4-[4-(phenylsulphonamido)phenyl]butyric acid. (1.03g, 58%) mp. 128-130°C.

$C_{16}H_{17}NO_4S$

| | | | | |
|---|---|---|---|---|
| Found: | C 60.19 | H 5.47 | N 4.31 | S 10.00 |
| Requires: | C 60.17 | H 5.37 | N 4.39 | S 10.04 |

Examples 2 to 4

Using the method described in Example 1, the following compounds were prepared. In each case, the appropriate phenylsulphonyl chloride was obtained from a commercial source.

2. 4-[4-(4-Chlorophenylsulphonamido)phenyl]butyric acid;
melting point 150-151°C.
Found: C 54.59 H 4.72 N 3.72 S 8.83 Cl 9.88
Requires: C 54.31 H 4.56 N 3.96 S 9.06 Cl 10.02

3. 4-[4-(4-Methoxyphenylsulphonamido)phenyl]butyric acid;
melting point 176-177°C
Found: C 58.67 H 5.57 N 3.95 S 9.28
Requires: C 58.40 H 5.48 N 4.01 S 9.18

4. 4-[4-(4-Bromophenylsulphonamido)phenyl]butyric acid.
melting point 168-169°C
Found: C 48.17 H 4.13 N 3.40 S 7.92 Br 20.33
Requires: C 48.25 H 4.05 N 3.52 S 8.05 Br 20.06.

## Claims

### Claims for the following Contracting States : AT, BE, CH, DE, FR, GB, IT, LI, LU, NL, SE

1. A compound of the formula (I):

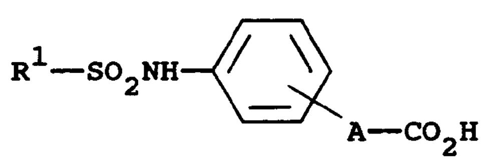

(I)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy, nitro and trifluoromethyl; and A is an acyclic hydrocarbon group having from 2 to 4 linear carbon atoms provided that when A is CH=CH, $(CH_2)_2$ or $(CH_2)_3$, $R^1$ is other than 4-methylphenyl.

2. A compound according to claim 1 wherein A is selected from $(CH_2)_3$, $(CH_2)_4$, $(CH_2)_2C(CH_3)_2$ and $(CH_2)_3C(CH_3)_2$.

3. A compound according to claim 1 or claim 2 wherein $R^1$ is unsubstituted phenyl, 4-chlorophenyl or 4-bromophenyl.

4. A compound according to any one of claims 1 to 3 wherein the group A-$CO_2H$ is <u>para</u> with respect to the group $R^1SO_2NH$.

5. A compound of formula (I) which is
4-[4-(phenylsulphonamido)phenyl]butyric acid;
4-[4-(4-chlorophenylsulphonamido)phenyl]butyric acid;
4-[4-(4-bromophenylsulphonamido)phenyl]butyric acid;
4-[4-(4-methoxyphenylsulphonamido)phenyl]butyric acid;
2,2-dimethyl-4-[4-(4-methylphenylsulphonamido)phenyl]butyric acid;
2,2-dimethyl-4-[4-(4-chlorophenylsulphonamido)phenyl]butyric acid;
2,2-dimethyl-4-[4-(4-bromophenylsulphonamido)phenyl]butyric acid;
2,2-dimethyl-4-[4-phenylsulphonamido)phenyl]butyric acid; or
2,2-dimethyl-4-[4-(4-methoxyphenylsulphonamido)phenyl]butyric acid;
or a pharmaceutically acceptable salt thereof.

6. A compound of the formula (IA):

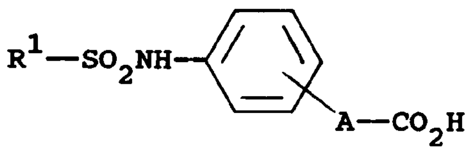

(IA)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy, nitro and trifluoromethyl; and A is an acyclic hydrocarbon group having from 2 to 4 linear carbon atoms, for use in medicine.

7. A compound of the formula (IA) as defined in claim 6 for use in the treatment of a thromboxane-$A_2$ mediated disease.

8. A pharmaceutical composition comprising a compound of the formula (IA) as defined in claim 6 and a pharmaceutically acceptable carrier.

9. A process for preparing a compound as defined in any one of claims 1 to 5 which process comprises the reaction of a compound of the formula (II):

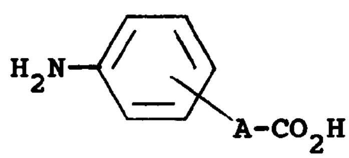

(II)

or a carboxylate salt, amide or ester thereof, with a compound $R^1SO_2L$ where L is a leaving group displaceable by amino.

**Claims for the following Contracting States : ES, GR**

1. A process for preparing a compound of the formula (I):

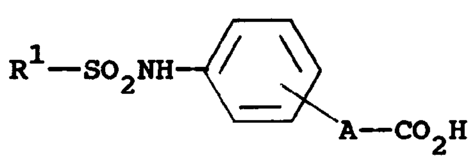

(I)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy, nitro and trifluoromethyl; and A is an acyclic hydrocarbon group having from 2 to 4 linear carbon atoms provided that when A is CH=CH, $(CH_2)_2$ or $(CH_2)_3$, $R^1$ is other than 4-methylphenyl, which comprises reaction of a compound of the formula (II):

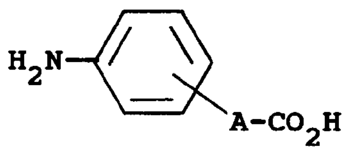

(II)

or a carboxylate salt, amide or ester thereof, with a compound $R^1SO_2L$ where L is a leaving group displaceable by amino, and $R^1$ is as described in formula (I).

2. A process according to claim 1 in which L is halogen.

3. A process according to claim 2 in which L is chlorine.

4. A process according to claim 3 in which the compound of formula (I) prepared is selected from
4-[4-(phenylsulphonamido)phenyl]butyric acid;
4-[4-(4-chlorophenylsulphonamido)phenyl]butyric acid;
4-[4-(4-bromophenylsulphonamido)phenyl]butyric acid;
4-[4-(4-methoxyphenylsulphonamido)phenyl]butyric acid;
2,2-dimethyl-4-[4-(4-methylphenylsulphonamido)phenyl]butyric acid;
2,2-dimethyl-4-[4-(4-chlorophenylsulphonamido)phenyl]butyric acid;
2,2-dimethyl-4-[4-(4-bromophenylsulphonamido)phenyl]butyric acid;
2,2-dimethyl-4-[4-phenylsulphonamido)phenyl]butyric acid; and
2,2-dimethyl-4-[4-(4-methoxyphenylsulphonamido)phenyl]butyric acid;
or a pharmaceutically acceptable salt thereof.

5. A process for preparing a pharmaceutical composition which comprises bringing into association a compound of formula (IA):

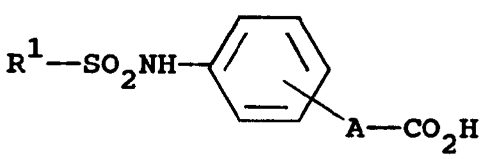

(IA)

and salts, esters and amides thereof, wherein $R^1$ is phenyl optionally substituted by one or more substituents chosen from halogen, $C_{1-4}$alkyl, $C_{1-6}$acyl, $C_{1-4}$alkoxy, nitro and trifluoromethyl; and A is an acyclic hydrocarbon group having from 2 to 4 linear carbon atoms, and a pharmaceutically acceptable carrier.

6. A process according to claim 5 in which the compound of formula (I) is selected from
4-[4-(phenylsulphonamido)phenyl]butyric acid;
4-[4-(4-chlorophenylsulphonamido)phenyl]butyric acid;
4-[4-(4-bromophenylsulphonamido)phenyl]butyric acid;
4-[4-(4-methoxyphenylsulphonamido)phenyl]butyric acid;
2,2-dimethyl-4-[4-(4-methylphenylsulphonamido)phenyl]butyric acid;
2,2-dimethyl-4-[4-(4-chlorophenylsulphonamido)phenyl]butyric acid;
2,2-dimethyl-4-[4-(4-bromophenylsulphonamido)phenyl]butyric acid;
2,2-dimethyl-4-[4-phenylsulphonamido)phenyl]butyric acid; and
2,2-dimethyl-4-[4-(4-methoxyphenylsulphonamido)phenyl]butyric acid;
or a pharmaceutically acceptable salt thereof.

## Patentansprüche

### Patentansprüche für folgende Vertragsstaaten : AT, BE, CH, DE, FR, GB, IT, LI, LU, NL, SE

1. Verbindung der Formel (I):

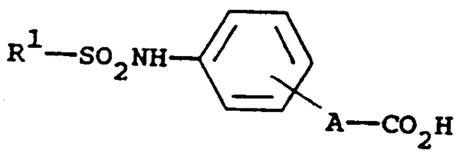

(I)

und Salze, Ester und Amide davon, wobei $R^1$ eine Phenylgruppe darstellt, die gegebenenfalls mit einem oder mehreren Substituenten, ausgewählt aus Halogenatomen, $C_{1-4}$-Alkyl-, $C_{1-6}$-Acyl- oder $C_{1-4}$-Alkoxyresten, Nitro- und Trifluormethylgruppen substituiert ist und A einen acyclischen Kohlenwasserstoffrest mit 2 bis 4 Kohlenstoffatomen in linearer Anordnung bedeutet, mit der Maßgabe, daß, wenn A CH=CH, $(CH_2)_2$ oder $(CH_2)_3$ bedeutet, $R^1$ keine 4-Methylphenylgruppe ist.

2. Verbindung nach Anspruch 1, wobei A ausgewählt ist aus $(CH_3)_3$, $(CH_2)_4$, $(CH_2)_2C(CH_3)_2$ und $(CH_2)_3C(CH_3)_2$.

3. Verbindung nach Anspruch 1 oder Anspruch 2, wobei $R^1$ eine unsubstituierte Phenyl-, 4-Chlorphenyl- oder 4-Bromphenylgruppe bedeutet.

4. Verbindung nach einem der Ansprüche 1 bis 3, wobei die Gruppe A-$CO_2H$, bezogen auf die Gruppe $R^1SO_2NH$, in para-Stellung vorliegt.

5. Verbindung der Formel (I), nämlich
4-[4-(Phenylsulfonamido)phenyl]buttersäure;
4-[4-(4-Chlorphenylsulfonamido)phenyl]buttersäure;
4-[4-(4-Bromphenylsulfonamido)phenyl]buttersäure;
4-[4-(4-Methoxyphenylsulfonamido)phenyl]buttersäure;
2,2-Dimethyl-4-[4-(4-methylphenylsulfonamido)phenyl]buttersäure;
2,2-Dimethyl-4-[4-(4-chlorphenylsulfonamido)phenyl]buttersäure;
2,2-Dimethyl-4-[4-(4-bromphenylsulfonamido)phenyl]buttersäure;
2,2-Dimethyl-4-[4-(phenylsulfonamido)phenyl]buttersäure;
oder
2,2-Dimethyl-4-[4-(4-methoxyphenylsulfonamido)phenyl]buttersäure;
oder ein pharmazeutisch verträgliches Salz davon.

6. Verbindung der Formel (IA):

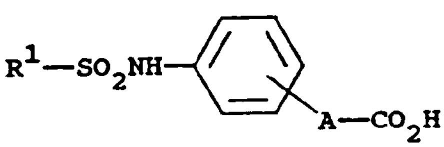

(IA)

und Salze, Ester und Amide davon, wobei $R^1$ eine Phenylgruppe darstellt, die gegebenenfalls mit einem oder mehreren Substituenten, ausgewählt aus Halogenatomen, $C_{1-4}$-Alkyl-, $C_{1-6}$-Acyl-, $C_{1-4}$-Alkoxyresten, Nitro- und Trifluormethylgruppen, substituiert ist; und A einen acyclischen Kohlenwasserstoffrest mit 2 bis 4 Kohlenstoffatomen in linearer Anordnung bedeutet, zur Verwendung in der Medizin.

7. Verbindung der Formel (IA), definiert wie in Anspruch 6, zur Verwendung bei der Behandlung einer durch Thromboxan-$A_2$ vermittelten Erkrankung.

8. Arzneimittel, umfassend eine Verbindung der Formel (IA), wie in Anspruch 6 definiert, und einen pharmazeutisch verträglichen Träger.

9. Verfahren zur Herstellung einer Verbindung nach einem der Ansprüche 1 bis 5, wobei das Verfahren die Umsetzung einer Verbindung der Formel (II):

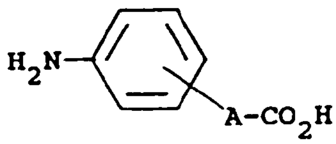

(II)

oder eines Carbonsäuresalzes, Amids oder Esters davon, mit einer Verbindung der Formel $R^1SO_2L$ umfaßt, in der L eine Austrittsgruppe bedeutet, die durch eine Aminogruppe ersetzbar ist.

**Patentansprüche für folgende Vertragsstaaten : ES, GR**

1. Verfahren zur Herstellung einer Verbindung der Formel (I):

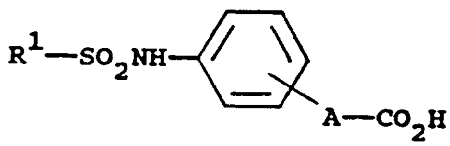

(I)

und Salzen, Estern und Amiden davon, wobei $R^1$ eine Phenylgruppe darstellt, die gegebenenfalls mit einem oder mehreren Substituenten, ausgewählt aus Halogenatomen, $C_{1-4}$-Alkyl-, $C_{1-6}$-Acyl- oder $C_{1-4}$-Alkoxyresten, Nitro- und Trifluormethylgruppen substituiert ist und A einen acyclischen Kohlenwasserstoffrest mit 2 bis 4 Kohlenstoffatomen in linearer Anordnung bedeutet, mit der Maßgabe, daß, wenn A CH=CH, $(CH_2)_2$ oder $(CH_2)_3$ bedeutet, $R^1$ keine 4-Methylphenylgruppe ist, wobei das Verfahren die Umsetzung einer Verbindung der Formel (II):

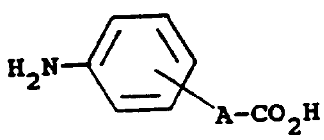

(II)

oder eines Carbonsäuresalzes, Amids oder Esters davon, mit einer Verbindung der Formel $R^1SO_2L$ umfaßt, in der L eine Austrittsgruppe bedeutet, die durch eine Aminogruppe ersetzbar ist und $R^1$ die für Formel (I) beschriebene Bedeutung besitzt.

2. Verfahren nach Anspruch 1, wobei L ein Halogenatom bedeutet.

3. Verfahren nach Anspruch 2, wobei L ein Chloratom bedeutet.

4. Verfahren nach Anspruch 3, wobei die hergestellte Verbindung der Formel (I) ausgewählt ist aus
4-[4-(Phenylsulfonamido)phenyl]buttersäure;
4-[4-(4-Chlorphenylsulfonamido)phenyl]buttersäure;
4-[4-(4-Bromphenylsulfonamido)phenyl]buttersäure;
4-[4-(4-Methoxyphenylsulfonamido)phenyl]buttersäure;
2,2-Dimethyl-4-[4-(4-methylphenylsulfonamido)phenyl]buttersäure;
2,2-Dimethyl-4-[4-(4-chlorphenylsulfonamido)phenyl]buttersäure;
2,2-Dimethyl-4-[4-(4-bromphenylsulfonamido)phenyl]buttersäure;
2,2-Dimethyl-4-[4-(phenylsulfonamido)phenyl]buttersäure;
und
2,2-Dimethyl-4-[4-(4-methoxyphenylsulfonamido)phenyl]buttersäure;
oder einem pharmazeutisch verträglichen Salz davon.

5. Verfahren zur Herstellung eines Arzneimittels, umfassend das Zusammenbringen einer Verbindung der Formel (IA):

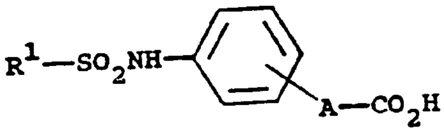

(IA)

und Salzen, Estern und Amiden davon, wobei $R^1$ eine Phenylgruppe darstellt, die gegebenenfalls mit einem oder mehreren Substituenten, ausgewählt aus Halogenatomen, $C_{1-4}$-Alkyl-, $C_{1-6}$Acyl-, $C_{1-4}$Alkoxyresten, Nitro- und Trifluormethylgruppen, substituiert ist; und A einen acyclischen Kohlenwasserstoffrest mit 2 bis 4 Kohlenstoffatomen in linearer Anordnung bedeutet, mit einem pharmazeutisch verträglichen Träger.

6. Verfahren nach Anspruch 5, wobei die Verbindung der Formel I ausgewählt ist aus:
4-[4-(Phenylsulfonamido)phenyl]buttersäure;
4-[4-(4-Chlorphenylsulfonamido)phenyl]buttersäure;
4-[4-(4-Bromphenylsulfonamido)phenyl]buttersäure;
4-[4-(4-Methoxyphenylsulfonamido)phenyl]buttersäure;
2,2-Dimethyl-4-[4-(4-methylphenylsulfonamido)phenyl]buttersäure;
2,2-Dimethyl-4-[4-(4-chlorphenylsulfonamido)phenyl]buttersäure;
2,2-Dimethyl-4-[4-(4-bromphenylsulfonamido)phenyl]buttersäure;
2,2-Dimethyl-4-[4-(phenylsulfonamido)phenyl]buttersäure;
und
2,2-Dimethyl-4-[4-(4-methoxyphenylsulfonamido)phenyl]buttersäure;
oder einem pharmazeutisch verträglichen Salz davon.

## Revendications

### Revendications pour les Etats contractants suivants : AT, BE, CH, DE, FR, GB, IT, LI, LU, NL, SE

1. Composé de formule (I) :

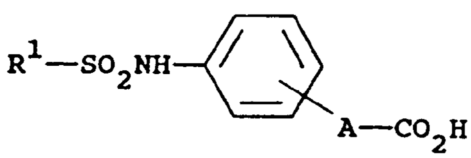

(I)

et sels, esters et amides de celui-ci, dans laquelle $R^1$ est un groupe phényle éventuellement substitué par un ou plusieurs substituants choisis parmi un atome d'halogène, un groupe alkyle en $C_{1-4}$ , acyle en $C_{1-6}$ , alcoxy en $C_{1-4}$ , nitro et trifluorométhyle ; et A est un groupe hydrocarboné acyclique ayant de 2 à 4 atomes de carbone linéaire à condition que lorsque A est un groupe CH = CH, $(CH_2)_2$ ou $(CH_2)_3$ , $R^1$ soit autre qu'un groupe 4-méthylphényle.

2. Composé suivant la revendication 1, dans lequel A est choisi parmi les groupes $(CH_2)_3$ , $(CH_2)_4$ , $(CH_2)_2C(CH_3)_2$ et $(CH_2)_3C(CH_3)_2$ .

3. Composé suivant la revendication 1 ou la revendication 2, dans lequel $R^1$ est un groupe phényle non-substitué, 4-chlorophényle ou 4-bromophényle.

4. Composé suivant l'une quelconque des revendications 1 à 3, dans lequel le groupe $A\text{-}CO_2H$ est en para par rapport au groupe $R^1SO_2NH$.

5. Composé de formule (I) qui est :
l'acide 4-[4-(phénylsulfonamido)phényl]butyrique,
l'acide 4- [4-(4-chlorophénylsulfonamido)phényl]butyrique,
l'acide 4-[4-(4-bromophénylsulfonamido)phényl]butyrique,

l'acide 4-[4-(4-méthoxyphénylsulfonamido)phényl]butyrique,
l'acide 2,2-diméthyl-4-[4-(4-méthylphénylsulfonamido)phényl]butyrique,
l'acide 2,2-diméthyl-4-[4-(4-chlorophénylsulfonamido)phényl]butyrique,
l'acide 2,2-diméthyl-4-[4-(4-bromophénylsulfonamido)phényl]butyrique,
l'acide 2,2-diméthyl-4- (4-(4-phénylsulfonamido)phényl]butyrique, ou
l'acide 2,2-diméthyl-4-(4-(4-méthoxyphénylsulfonamido)phényl]butyrique,
ou un sel de ceux-ci acceptable du point de vue pharmaceutique.

6. Composé de formule (IA) :

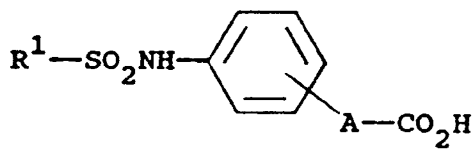

(IA)

et sels, esters et amides de celui-ci, dans laquelle $R^1$ est un groupe phényle éventuellement substitué par un ou plusieurs substituants choisis parmi un atome d'halogène, un groupe alkyle en $C_{1-4}$, acyle en $C_{1-6}$, alcoxy en $C_{1-4}$, nitro et trifluorométhyle ; et A est un groupe hydrocarboné acyclique ayant de 2 à 4 atomes de carbone linéaire, utile en médecine.

7. Composé de formule (IA) tel que défini dans la revendication 6, utile dans le traitement d'une maladie médiatisée par la thromboxane $A_2$.

8. Composition pharmaceutique comprenant un composé de formule (IA) tel que défini dans la revendication 6 et un support acceptable du point de vue pharmaceutique.

9. Procédé pour préparer un composé tel que défini dans l'une quelconque des revendications 1 à 5, lequel procédé comprend la réaction d'un composé de formule (II) :

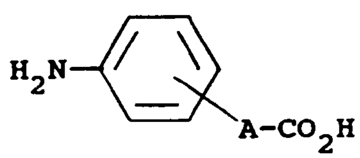

(II)

ou d'un sel carboxyle, d'un amide ou d'un ester de celui-ci, avec un composé $R^1SO_2L$ dans lequel L est un groupe mobile pouvant être déplacé par la fonction amino.

**Revendications pour les Etats contractants suivants : ES, GR**

1. Procédé pour préparer un composé de formule (I) :

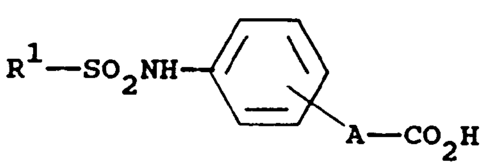

(I)

et des sels, esters et amides de celui-ci, dans laquelle $R^1$ est un groupe phényle éventuellement substitué par un ou plusieurs substituants choisis parmi un atome d'halogène, un groupe alkyle en $C_{1-4}$, acyle en $C_{1-6}$, alcoxy en $C_{1-4}$, nitro et trifluorométhyle ; et A est un groupe hydrocarboné acyclique ayant de 2 à 4 atomes de carbone linéaire, à condition que lorsque A est un groupe CH = CH, $(CH_2)_2$, ou $(CH_2)_3$, $R^1$ soit autre que le groupe 4-méthylphényle, qui comprend la réaction d'un composé de formule (II) :

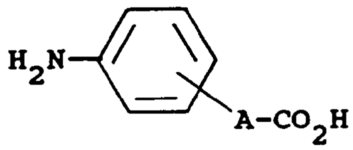

(II)

ou d'un sel carboxylique, d'un amide ou d'un ester de celui-ci, avec un composé $R^1SO_2L$ dans lequel L est un groupe mobile pouvant être déplacé par la fonction amino, et $R^1$ est tel que défini dans la formule (I).

2. Procédé suivant la revendication 1, dans lequel L est un atome d'halogène.

3. Procédé suivant la revendication 2, dans lequel L est un atome de chlore.

4. Procédé suivant la revendication 3, dans lequel le composé de formule (I) préparé est choisi parmi :
l'acide 4-[4-(phénylsulfonamido)phényl]butyrique,
l'acide 4-[4-(4-chlorophénylsulfonamido)phényl]butyrique,
l'acide 4-[4-(4-bromophénylsulfonamido)phényl]butyrique,
l'acide 4-[4-(4-méthoxyphénylsulfonamido)phényl]butyrique,
l'acide 2,2-diméthyl-4-[4-(4-méthylphénylsulfonamido)phényl]butyrique,
l'acide 2,2-diméthyl-4-[4-(4-chlorophénylsulfonamido)phényl]butyrique,
l'acide 2,2-diméthyl-4-[4-(4-bromophénylsulfonamido)phényl]butyrique,
l'acide 2,2-diméthyl-4-[4-(4-phénylsulfonamido)phényl]butyrique, et
l'acide 2,2-diméthyl-4-[4-(4-méthoxyphénylsulfonamido)phényl]butyrique,
ou un sel de ceux-ci acceptable du point de vue pharmaceutique.

5. Procédé pour préparer une composition pharmaceutique qui comprend la mise en association d'un composé de formule (IA) :

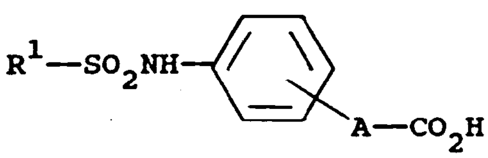

(IA)

et des sels, esters et amides de celui-ci, dans laquelle $R^1$ est un groupe phényle éventuellement substitué par un ou plusieurs substituants choisis parmi un atome d'halogène, un groupe alkyle en $C_{1-4}$ , acyle en $C_{1-6}$, alcoxy en $C_{1-4}$, nitro et trifluorométhyle ; et A est un groupe hydrocarboné acyclique ayant de 2 à 4 atomes de carbone linéaire, et d'un support acceptable du point de vue pharmaceutique.

6. Procédé suivant la revendication 5, dans lequel le composé de formule (I) est choisi parmi :
l'acide 4-[4-(phénylsulfonamido)phényl]butyrique,
l'acide 4-[4-(4-chlorophénylsulfonamido)phényl]butyrique,
l'acide 4-[4-(4-bromophénylsulfonamido)phényl]butyrique,
l'acide 4-[4-(4-méthoxyphénylsulfonamido)phényl]butyrique,
l'acide 2,2-diméthyl-4-[4-(4-méthylphénylsulfonamido)phényl]butyrique,
l'acide 2,2-diméthyl-4-[4-(4-chlorophénylsulfonamido)phényl]butyrique,
l'acide 2,2-diméthyl-4-[4-(4-bromophénylsulfonamido)phényl]butyrique,
l'acide 2,2-diméthyl-4-[4-(4-phénylsulfonamido)phényl]butyrique, et
l'acide 2,2-diméthyl-4- [4-(4-méthoxyphénylsulfonamido)phényl]butyrique,
ou un sel de ceux-ci acceptable du point de vue pharmaceutique.